(19) Europäisches Patentamt
European Patent Office
Office européen des brevets

(11) Veröffentlichungsnummer: **0 034 718**
**B1**

(12) # EUROPÄISCHE PATENTSCHRIFT

(45) Veröffentlichungstag der Patentschrift: **23.11.83**

(51) Int. Cl.³: **C 07 C 143/70**, C 07 C 63/10, C 07 C 63/06, C 07 C 139/00, C 07 C 51/58, C 07 C 51/00

(21) Anmeldenummer: **81100612.1**

(22) Anmeldetag: **28.01.81**

(54) **Verfahren zur Herstellung von aromatischen Sulfonsäurehalogeniden.**

(30) Priorität: **08.02.80 DE 3004693**

(43) Veröffentlichungstag der Anmeldung: **02.09.81 Patentblatt 81/35**

(45) Bekanntmachung des Hinweises auf die Patenterteilung: **23.11.83 Patentblatt 83/47**

(84) Benannte Vertragsstaaten: **BE CH DE FR GB IT LI**

(56) Entgegenhaltungen:
**EP - A - 0 009 205**
**CH - A - 574 896**
**DE - C - 574 836**
**US - A - 3 686 301**

(73) Patentinhaber: **BAYER AG, Zentralbereich Patente, Marken und Lizenzen, D-5090 Leverkusen 1, Bayerwerk (DE)**

(72) Erfinder: **Blank, Heinz Ulrich, Dr., Am Geusfelde 35, D-5068 Odenthal (DE)**
Erfinder: **Wolters, Erich, Dr., Streffenweg 22, D-5162 Niederzier (DE)**
Erfinder: **Langenfeld, Norbert, Dr., Hofstrasse 53, D-5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur Herstellung von aromatischen Sulfonsäurehalogeniden

Die Erfindung betrifft ein Verfahren zur gleichzeitigen Herstellung von aromatischen Sulfonsäurehalogeniden und aromatischen Carbonsäurehalogeniden oder den zugehörigen Carbonsäuren durch Umsetzung von aromatischen Sulfonsäuren mit aromatischen Trihalogenmethyl-Verbindungen in Gegenwart einer Brönsted- oder Lewis-Säure.

Es ist aus der US-Patentschrift 3 686 301 bekannt, 3,5-Dichlorsulfo-benzoylchlorid durch Reaktion von 180°C heisser 3,5-Disulfo-benzoesäure mit der dreifach molaren Menge Benzotrichlorid zu erhalten, wobei das Benzotrichlorid während eines Zeitraumes von 3 Stunden zugegeben wird und die Temperatur während der Zugabe langsam auf 130°C reduziert wird und das Gemisch eine weitere Stunde bei 130°C gerührt wird.

Weiterhin ist aus DRP 574 836 bekannt, die Natriumsalze aromatischer Sulfonsäuren mit Benzotrichlorid zu dem entsprechenden aromatischen Sulfonsäurechlorid umzusetzen. Diese DRP enthält weiterhin den Hinweis, dass die bekannte Umsetzung freier Carbonsäuren mit Benzotrichlorid in Gegenwart von Zinkchlorid zur Herstellung der Carbonsäurechloride auf freie organische Sulfonsäuren nicht übertragbar ist.

Es wurde nun ein Verfahren zur gleichzeitigen Herstellung aromatischer Sulfonsäurehalogenide und aromatischer Carbonsäurehalogenide oder der zugehörigen Carbonsäuren durch Umsetzung aromatischer Sulfonsäuren oder Sulfonsäureanhydride mit aromatischen Trihalogenmethyl-Verbindungen gefunden, das dadurch gekennzeichnet ist, dass man mindestens ein halbes Äquivalent einer aromatischen Trihalogenmethyl-Verbindung pro Äquivalent Sulfonsäure in Gegenwart einer Brönsted- oder Lewis-Säure bei einer Temperatur von 30 bis 130°C umsetzt.

Als aromatische Sulfonsäuren können im erfindungsgemässen Verfahren beispielsweise solche der allgemeinen Formel

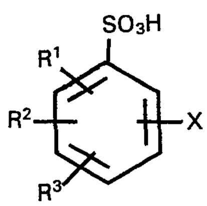

(I)

eingesetzt werden, in der

X für Wasserstoff, 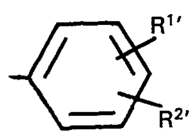

oder -Y- 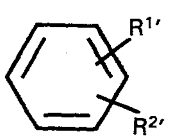

steht, wobei Y für -O-, $-SO_2-$, -N=N- oder $-CH_2-$ steht, und

$R^1$, $R^2$, $R^3$, $R^{1'}$ und $R^{2'}$ unabhängig voneinander Wasserstoff, Alkyl, Cycloalkyl, Aryl, Aralkyl, Hydroxy, Alkoxy, Alcyloxy, Halogen, Isocyanato, Acylamino, N-Carbalkoxyamino, N-Carbaryloxyamino, Nitro, die Sulfonsäuregruppe, die Carbonsäuregruppe, die Carbonsäureestergruppe oder die Harnstoffgruppe bedeuten, und weiterhin zwei der Reste $R^1$, $R^2$ oder $R^3$, wenn sie benachbart sind, Teil eines kondensierten, gegebenenfalls durch eine Sulfonsäuregruppe substituierten cycloaliphatischen oder aromatischen Ringes sein können.

Anstelle der genannten Sulfonsäuren können auch die entsprechenden Sulfonsäureanhydride eingesetzt werden.

Als aromatische Trihalogenmethyl-Verbindungen für das erfindungsgemässe Verfahren können beispielsweise solche der allgemeinen Formel

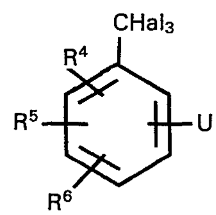

(II)

eingesetzt werden, in der

U für Wasserstoff, 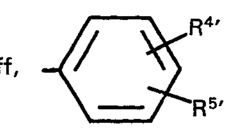

oder -V- 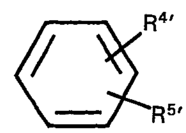

steht, wobei V für -O-, $-SO_2-$, -N=N- oder $-CH_2-$ steht, und

$R^4$, $R^5$, $R^6$, $R^{4'}$ und $R^{5'}$ unabhängig voneinander Wasserstoff, Alkyl, Cycloalkyl, Aryl, Aralkyl, Alkoxy, Aryloxy, $CHal_3$, Halogen, Isocyanato, N-Carbalkoxyamino oder N-Carbaryloxyamino bedeuten und weiterhin zwei der Reste $R^4$, $R^5$ oder $R^6$, wenn sie benachbart sind, Teil eines kondensierten, gegebenenfalls durch $CHal_3$ substituierten cycloaliphatischen oder aromatischen Ringes sein können und

Hal für Chlor oder Brom steht.

Als Alkyl seien beispielsweise geradkettige oder verzweigte Kohlenwasserstoffreste mit 1 bis 6, bevorzugt 1 bis 4, besonders bevorzugt 1 bis 2 Kohlenstoffatomen genannt, wie Methyl, Ethyl, Propyl, Isopropyl, Butyl, Isobutyl, Pentyl oder Hexyl.

Als Cycloalkyl seien beispielsweise Cyclopentyl und Cyclohexyl, bevorzugt Cyclohexyl, genannt.

Als Aryl seien beispielsweise aromatische carbocyclische Reste wie Phenyl, Naphthyl oder Anthryl, bevorzugt Phenyl, genannt.

Als Aralkyl seien beispielsweise Kohlenwasserstoffreste mit 1 bis 6 Kohenstoffatomen im aliphatischen Teil und 6 bis 14 Kohlenstoffatomen im aromatischen Teil genannt, wie Benzyl, β-Phenyl-ethyl, Naphthylmethyl, Naphthylethyl, Anthrylmethyl, γ-Phenyl-propyl und β-Phenyl-n-hexyl. Bevorzugtes Aralkyl ist Benzyl.

Als Alkoxy seien beispielsweise geradkettige oder verzweigte Reste von aliphatischen Alkoholen von 1 bis 6, bevorzugt 1 bis 4, besonders 1 bis 2 Kohlenstoffatomen genannt, wie Methoxy, Ethoxy, Propoxy, Isopropyloxy, Butoxy, Isobutyloxy, Pentyloxy und Hexyloxy.

Als Aryloxy seien Reste von aromatischen Hydroxylverbindungen mit 6 bis 14 Kohlenstoffatomen genannt, wie Phenoxy, Naphthyloxy und Anthryloxy, bevorzugt Phenoxy.

Als Halogen seien beispielsweise Fluor, Chlor, Brom und Jod, bevorzugt Fluor, Chlor und Brom, besonders bevorzugt Chlor und Fluor, genannt.

Für den Fall, dass zwei der Reste $R^1$ bis $R^3$ oder $R^4$ bis $R^6$, wenn sie benachbart sind, Teile eines kondensierten cycloaliphatischen oder aromatischen Ringes sein können, seien unter Einschluss des Benzolkerns der Formel (I) bzw. der Formel (II) das Indan-, Inden-, Tetrahydronaphthalin- und Naphthalin-System verstanden. Die bevorzugten kondensierten Ringsysteme sind das Naphthalin- und das Tetrahydronaphthalin-System. Besonders bevorzugt ist das Naphthalin-System.

Als bevorzugte aromatische Sulfonsäuren für das erfindungsgemässe Verfahren seien beispielsweise solche der Formel

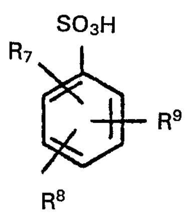 (III)

genannt, in der

$R^7$, $R^8$ und $R^9$ unabhängig voneinander Wasserstoff, $C_1$-$C_4$-Alkyl, Phenyl, Benzyl, Methoxy, Ethoxy, Phenoxy, Fluor, Chlor, Brom, Nitro oder die Sulfonsäuregruppe bedeuten und in der weiterhin zwei der Reste $R^7$ bis $R^9$, wenn sie benachbart sind, Teil eines kondensierten cycloaliphatischen oder aromatischen 6-Ringes sein können.

Als aromatische Sulfonsäuren für das erfindungsgemässe Verfahren seien besonders bevorzugt solche der allgemeinen Formel

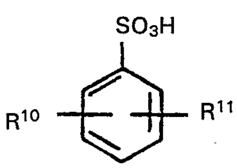 (IV)

genannt, in der

$R^{10}$ und $R^{11}$ unabhängig voneinander Wasserstoff, Methyl, Ethyl, Chlor oder Fluor bedeuten und in der $R^{10}$ und $R^{11}$, wenn sie benachbart sind, Teil eines kondensierten cycloaliphatischen oder aromatischen 6-Ringes sein können.

Als aromatische Sulfonsäuren für das erfindungsgemässe Verfahren seien beispielsweise genannt: Benzolsulfonsäure, 2-, 3- und 4-Toluolsulfonsäure, 2-, 3- und 4-Chlor-benzolsulfonsäure, 2,5- und 3,4-Dichlor-benzolsulfonsäure, Benzol-1,3-disulfonsäure, 2-Chlor-toluol-4-sulfonsäure, 4-Chlor-toluol-2--sulfonsäure, Toluol-2,4-disulfonsäure, 1-Acetylamino-benzol-4-sulfonsäure, 4-Carbomethoxyamino-benzolsulfonsäure, 1- und 2-Naphthalinsulfonsäure, Naphthalin-1,5-, -2,6- und -2,7-disulfonsäure, 4-Biphenyl-sulfonsäure, 4,4'-Biphenyl-disulfonsäure, 4-Phenoxy-benzolsulfonsäure und Diphenylmethan-4-sulfonsäure.

Als aromatische Trihalogenmethyl-Verbindungen für das erfindungsgemässe Verfahren seien besonders aromatische Trichlormethyl-Verbindungen genannt. Trichlormethyl-Verbindungen sind beispielsweise solche der allgemeinen Formel

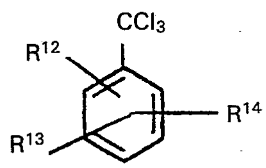 (V),

in der

$R^{12}$, $R^{13}$ und $R^{14}$ unabhängig voneinander Wasserstoff, Chlor, Trifluormethyl oder Trichlormethyl bedeuten und in der zwei der Reste $R^{12}$ bis $R^{14}$, wenn sie benachbart sind, Teil eines kondensierten cycloaliphatischen oder aromatischen 6-Ringes sein können.

Besonders bevorzugt werden im erfindungsgemässen Verfahren Trichlormethyl-Verbindungen der allgemeinen Formel

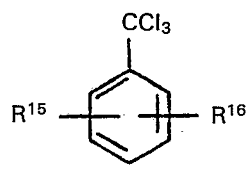 (VI)

eingesetzt, in der

$R^{15}$ und $R^{16}$ unabhängig voneinander Wasserstoff, Methyl, Chlor oder Trichlormethyl bedeuten und in der die Reste $R^{15}$ und $R^{16}$, wenn sie benachbart sind, Teil eines kondensierten cycloaliphatischen oder aromatischen 6-Ringes sein können.

Beispielsweise seien die folgenden Trichlormethyl-Verbindungen genannt: Benzotrichlorid, 1-Chlor-2-trichlormethylbenzol, 1-Chlor-4-trichlormethylbenzol, 2,4-Dichlor-1-trichlormethylbenzol, 1,3-Bis-(Trichlormethyl)-benzol, 1,4-Bis-(Trichlormethyl)-benzol, 1-Trichlormethyl-naphthalin, 2-Trichlormethyl-naphthalin, 4-Trichlormethyl-bisphenyl, 4,4'-Bis-(Trichlormethyl)-bisphenyl, 4-Trichlormethyl-diphenylether, 4-Trichlormethyl-diphenylmethan, Benzotribromid, 1-Brom-2-tribrommethylbenzol und 1-Brom-4-tribrommethylbenzol.

Im erfindungsgemässen Verfahren werden bevorzugt aromatische Sulfonsäuren der Formel (III) mit

aromatischen Trichlormethyl-Verbindungen der Formel (V), besonders bevorzugt Sulfonsäuren der Formel (IV) mit Trichlormethyl-Verbindungen der Formel (VI), umgesetzt.

Das erfindungsgemässe Verfahren wird in Gegenwart einer Brönsted- oder Lewis-Säure durchgeführt. Eine Brönsted-Säure ist allgemein dadurch gekennzeichnet, dass sie ein dissoziierbares Wasserstoffatom enthält; eine Lewis-Säure ist allgemein dadurch gekennzeichnet, dass sie eine Elektronenpaar-Lücke hat (siehe hierzu die Lehrbücher der theoretischen Chemie, beispielsweise H.A. Staab, Einführung in die theoretische organische Chemie, 3. Auflage, Seite 599 ff., Verlag Chemie, Weinheim 1962). Brönsted-Säuren für das erfindungsgemässe Verfahren sind beispielsweise Schwefelsäure, Pyroschwefelsäure, Phosphorsäure, Pyrophosphorsäure und die sauren Salze dieser Säuren, ferner Polyphosphorsäure, Fluorsulfonsäure, Chlorsulfonsäure und Bromsulfonsäure. Lewis-Säuren für das erfindungsgemässe Verfahren sind beispielsweise Aluminiumchlorid, Eisenchlorid, Zinkchlorid, Cadmiumchlorid, Quecksilber-(II)-chlorid, Berylliumchlorid, Magnesiumchlorid, Bortrifluorid, Bortrichlorid, Galliumchlorid, Titandichlorid, Titantetrachlorid, Zirkontetrachlorid, Nickelchlorid, Niobpentachlorid, Urantetrachlorid, Kupfer-(I)-chlorid, Kupfer-(II)-chlorid, Kobaltchlorid, Chromtrichlorid, Wismuttrichlorid, Antimontrichlorid, Antimonpentachlorid, Tellurtetrachlorid, Arsentrichlorid und Arsenpentachlorid. Die genannten Brönsted- und Lewis-Säuren können sowohl einzeln, als auch als Gemisch verschiedener Brönsted-Säuren, als Gemisch verschiedener Lewis-Säuren und als Gemisch von Brönsted- und Lewis-Säuren eingesetzt werden.

Im erfindungsgemässen Verfahren wird bevorzugt in Gegenwart von Schwefelsäure, Chlorsulfonsäure, Eisen-(III)-chlorid, Zinkchlorid oder einem Gemisch dieser Stoffe gearbeitet. Besonders bevorzugt wird in Gegenwart von Schwefelsäure oder Eisen-(III)-chlorid gearbeitet. Für das erfindungsgemässe Verfahren kann es weiterhin ausreichend sein, dass Sulfonierungsmittel, wie sie in technischen Sulfonsäuren aus dem Herstellungsprozess enthalten sind, beispielsweise Schwefelsäure oder Chlorsulfonsäure, gegenwärtig sind.

Die Brönsted- oder Lewis-Säure wird im erfindungsgemässen Verfahren beispielsweise in einer Menge von 0,01 bis 20 Mol%, bevorzugt von 0,1 bis 10 Mol-%, bezogen auf die eingesetzte Sulfonsäure, eingesetzt.

Das erfindungsgemässe Verfahren wird bei einer Temperatur von 30 bis 130°C, bevorzugt 40 bis 110°C, besonders bevorzugt 50 bis 95°C, durchgeführt.

Der Druck ist für die Durchführbarkeit des erfindungsgemässen Verfahrens nicht wesentlich, so dass bei Normaldruck, erhöhtem oder vermindertem Druck gearbeitet werden kann. Die bevorzugte Variante ist die bei Normaldruck.

Das erfindungsgemässe Verfahren kann mit oder ohne zusätzliches Reaktionsmedium durchgeführt werden. Geeignete Reaktionsmedien sind solche, die unter den Reaktionsbedingungen inert sind, beispielsweise Halogenkohlenwasserstoffe, wie Chloroform, Tetrachlorkohlenstoff, Trichlorethan, Tetrachlorethan, Pentachlorethan, Chlorbenzol, Dichlorbenzol, Chlortoluol oder Dichlortoluol. Als Reaktionsmedium kann weiterhin im Überschuss eingesetzte Trihalogenmethyl-Verbindung oder das im Reaktionsverlauf entstehende aromatische Carbonsäurechlorid dienen. Die bevorzugte Variante ist die ohne zusätzliches Lösungsmittel.

Zum vollständigen Umsatz der aromatischen Sulfonsäure im erfindungsgemässen Verfahren ist es erforderlich, mindestens ein halbes Äquivalent der aromatischen Trihalogenmethyl-Verbindung pro Äquivalent Sulfonsäure einzusetzen. Dabei entsteht aus der aromatischen Trihalogenmethyl-Verbindung die entsprechende Benzoesäure. Vorzugsweise ist es jedoch auch möglich, die aromatische Trihalogenmethyl-Verbindung in grösserer als der halbäquivalenten Menge, bezogen auf die aromatische Sulfonsäure, beispielsweise in äquimolarer Menge, einzusetzen. In diesem Falle entsteht aus der aromatischen Trihalogenmethyl-Verbindung das entsprechende Benzoylhalogenid. Weiterhin ist es möglich, die aromatische Trihalogenmethyl-Verbindung im Überschuss, bezogen auf die aromatische Sulfonsäure, beispielsweise in einer Menge von bis zu 5 Äquivalenten pro Äquivalent Sulfonsäure, einzusetzen. Bei dieser letzteren Variante dient die überschüssige aromatische Trihalogenmethyl-Verbindung als Lösungs- bzw. Verdünnungsmittel. Ein noch grösserer Überschuss ist für das erfindungsgemässe Verfahren unkritisch, jedoch aus wirtschaftlichen Gründen weniger günstig.

Die Reaktion des erfindungsgemässen Verfahrens sei bei verschiedenen Äquivalenzverhältnissen von aromatischer Sulfonsäure und aromatischer Trihalogenmethyl-Verbindung am Beispiel der Umsetzung von Benzolsulfonsäure mit Benzotrichlorid durch die folgenden Reaktionsgleichungen dargestellt:

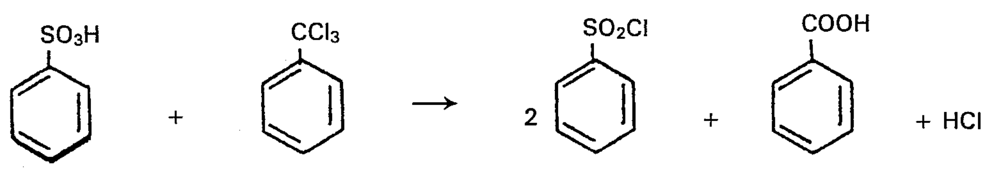

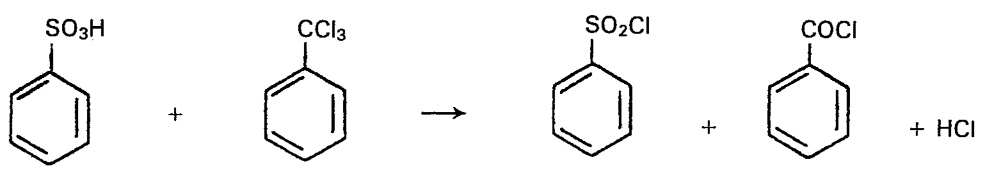

Im erfindungsgemässen Verfahren können demnach aromatische Sulfonsäurehalogenide der allgemeinen Formel

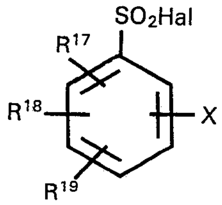

(VII).

hergestellt werden, in der

X für Wasserstoff,

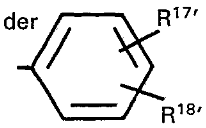

oder -Y-

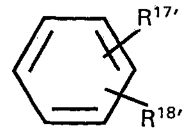

steht, wobei Y für -O-, $-SO_2-$, -N=N- oder $-CH_2-$ steht, und

$R^{17}$, $R^{18}$, $R^{19}$, $R^{17'}$ und $R^{18'}$ den Bedeutungen von $R^1$, $R^2$, $R^3$, $R^{1'}$ und $R^{2'}$ haben, wobei aber anstelle der Sulfonsäuregruppe $-SO_3H$ die Halogensulfonylgruppe $-SO_2Hal$ und anstelle der Carbonsäuregruppe $-CO_2H$ die Carbonsäurehalogenidgruppe -COHal erscheint, und

Hal für Chlor oder Brom steht.

Bevorzugt werden die folgenden Sulfochloride nach dem erfindungsgemässen Verfahren hergestellt. Benzolsulfochlorid, 2-, 3- und 4-Toluolsulfochlorid, 2-, 3- und 4-Chlorbenzolsulfochlorid, Benzol-1,3-disulfochlorid, 1- und 2-Naphthalinsulfochlorid.

Im erfindungsgemässen Verfahren wird die eingesetzte aromatische Trihalogenmethyl-Verbindung in die entsprechende aromatische Carbonsäure-Verbindung der allgemeinen Formel

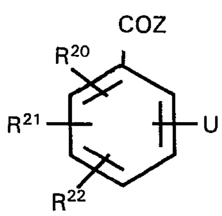

(VIII)

umgewandelt, in der

U für Wasserstoff,

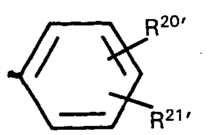

oder -V-

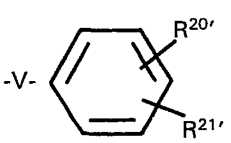

steht, wobei V für -O-, $-SO_2-$, -N=N- oder $-CH_2-$ steht,

$R^{20}$, $R^{21}$, $R^{22}$, $R^{20'}$ und $R^{21'}$ den Bedeutungsumfang von $R^4$, $R^5$, $R^6$, $R^{4'}$ und $R^{5'}$ haben, wobei jedoch anstelle der Trihalogenmethylgruppe $-CHal_3$ die Gruppe -COZ tritt, und

Z für Hydroxyl, Chlor oder Brom steht.

Das erfindungsgemässe Verfahren erlaubt somit neben der Herstellung des aromatischen Sulfonsäurehalogenids gleichzeitig die Herstellung einer aromatischen Carbonsäure, bzw. bevorzugt ihres zugehörigen Carbonsäurehalogenids. Hierbei besteht nicht nur eine breite Variationsmöglichkeit in bezug auf die Herstellung des aromatischen Sulfonsäurehalogenids, sondern ebenso durch die Auswahl der aromatischen Trihalogenmethyl-Verbindung im Rahmen der Formel (II) und durch die geschilderte Wahl der Äquivalenz zwischen aromatischer Sulfonsäure und aromatischer Trihalogenmethyl-Verbindung eine breite Variationsmöglichkeit bezüglich der gleichzeitigen Herstellung entweder einer aromatischen Carbonsäure oder ihres entsprechenden Carbonsäurehalogenids, sowie der Substitution dieser aromatischen Carbonsäure bzw. ihres Carbonsäurehalogenids.

Die Reihenfolge der Zugabe der Ausgangsstoffe im erfindungsgemässen Verfahren ist beliebig. So können beispielsweise hintereinander die aromatische Sulfonsäure, die aromatische Trihalogenmethyl-Verbindung und die Brönsted- oder Lewis-Säure zusammengegeben werden. Man kann jedoch auch die Brönsted- oder Lewis-Säure vorlegen und dann die aromatische Sulfonsäure und die aromatische Trihalogenmethyl-Verbindung hintereinander oder gleichzeitig getrennt oder als Gemisch zugeben.

Das erfindungsgemässe Verfahren kann absatzweise oder kontinuierlich durchgeführt werden. Bei der kontinuierlichen Durchführung kann beispielsweise in einem dem Fachmann bekannten Umlaufreaktor gearbeitet werden. Hierbei werden an einer Stelle des Umlaufreaktors über einen oder mehrere Einlässe die Ausgangsstoffe zugegeben, während das Reaktionsgemisch nach Durchlaufen des Umlaufreaktors an einer Stelle kurz vor dem Wiedererreichen der Zugabestellen zumindest teilweise dem Reaktor wieder entnommen wird. Das entnommene Reaktionsgemisch wird durch übliche Aufarbeitungsmethoden, beispielsweise durch fraktionierte Destillation, aufgearbeitet. Ein hierbei anfallender Rückstand, der die Brönsted- oder Lewis-Säure enthalten kann, kann zumindest teilweise dem Umlaufreaktor wieder zugesetzt werden.

Bei der absatzweisen Durchführung werden beispielsweise die aromatische Sulfonsäure und die aromatische Trihalogenmethyl-Verbindung in einer Rührapparatur mit der Brönsted- oder Lewis-Säure versetzt und unter Rühren auf die Reaktionstemperatur erhitzt, bis die Gasentwicklung beendet ist. Die Aufarbeitung erfolgt nach bekannten Methoden, beispielsweise durch fraktionierte Destillation, aber auch durch Extraktion oder andere Verfahren.

Der bei der Reaktion anfallende gasförmige Chlorwasserstoff kann in einer geeigneten Absorptionsapparatur beispielsweise als wässrige oder als alkoholische Salzsäure aufgefangen werden. Er kann jedoch auch in reiner Form komprimiert werden und in dieser Form weiterverwendet werden.

Die erfindungsgemäss herstellbaren aromatischen Sulfonsäurechloride und die gleichzeitig entstehenden aromatischen Carbonsäuren bzw. die entsprechenden aromatischen Carbonsäurehalogenide sind dem Fachmann als unentbehrliche Zwischenprodukte für eine Vielzahl von Synthesen, beispielsweise für Ester, Amide, Hydrazide und weitere Derivate der Sulfon- bzw. Carbonsäuren für Farbstoffe, Arzneimittel und andere Produkte, bekannt.

Das erfindungsgemässe Verfahren bietet gegenüber der Herstellung der Sulfonsäurechloride durch Chlorsulfonierung den Vorteil, dass keine Abfallsäuren entstehen. Gegenüber dem Verfahren mit Thionylchlorid oder Phosgen unter Dialkylformamid-Katalyse bietet es den Vorteil, dass keine schwer trennbaren Abgasgemische auftreten und dass keine potentiell carcinogenen Dialkylcarbamidsäurechloride gebildet werden können. Gegenüber der nicht katalysierten Reaktion von aromatischen Sulfonsäuren mit Trichlormethylaromaten hat das erfindungsgemässe Verfahren den Vorteil erheblich reduzierter Reaktionstemperaturen und -zeiten. Ein weiterer Vorteil besteht darin, dass die zu verwendende aromatische Trihalogenmethyl-Verbindung entsprechend ihrer Verfügbarkeit, der Trennbarkeit des Produktgemisches und dem Bedarf an der simultan entstehenden aromatischen Carbonsäure bzw. dem korrespondierenden aromatischen Sulfonsäurehalogenid weitgehend variiert werden kann. Gegebenenfalls kann die Menge der eingesetzten aromatischen Trihalogenmethyl-Verbindung gegenüber den Verfahren des Standes der Technik stark verringert werden.

*Beispiel 1 (Vergleichsbeispiel) und 2*

Je 158 g (1 Mol) Benzolsulfonsäure (98,5%ig, schwefelsäurefrei) und 195,5 g (116 ml, 1 Mol) Benzotrichlorid (98,5%ig) werden in einem Glaskolben mit Rührer, Innenthermometer und Rückflusskühler mit Blasenzähler auf 60°C erhitzt. Nach etwa 1/2 Stunde und Nachlassen der Gasentwicklung wird die erste Probe entnommen (0-Stunden-Probe). Dann wird in Beispiel 1 weiter erhitzt und nach den angegebenen Zeiten je eine Probe entnommen.

In Beispiel 2 werden nach der 0-Stunden-Probe 7,9 g (8 Mol-%) Schwefelsäure zugesetzt und in den jeweils gleichen Abständen Proben genommen und gaschromatographisch untersucht. Tabelle 1 enthält die Ausbeuten nach den entsprechenden Reaktionszeiten. Hierbei bedeuten $PhSO_2Cl$ Benzolsulfonsäurechlorid, PhCOCl Benzoylchlorid und $PhCCl_3$ Benzotrichlorid. Prozentangaben in allen Beispielen beziehen sich auf die theoretische Ausbeute.

Tabelle 1

| Beispiel | 1 | | | 2 | | |
|---|---|---|---|---|---|---|
| Reaktionszeit nach Ende der Gasentwicklung | $PhSO_2Cl$ % | PhCOCl % | $PhCCl_3$ % | $PhSO_2Cl$ % | PhCOCl % | $PhCCl_3$ % |
| 0 Stunden | 8 | 56 | 44 | 5 | 52 | 43 |
| 0,5 Stunden | 9 | 58 | 42 | 89 | 85 | 3 |
| 2 Stunden | 14 | 60 | 40 | 90 | 87 | 0 |
| 24 Stunden | 64 | 88 | 12 | | | |
| 48 Stunden | 81 | 94 | 6 | | | |
| 120 Stunden | 84 | 96 | 4 | | | |

*Beispiel 3 und 4 (Vergleichsbeispiele)*

158 g (1 Mol) Benzolsulfonsäure (98,5%ig, schwefelsäurefrei) werden in einem Glaskolben mit Rührer, Innenthermometer, Tropftrichter und Rückflusskühler vorgelegt und auf 60 bzw. 110°C erwärmt (siehe Tabelle 2). Während 3 Stunden werden 195,5 g (116 ml, 1 Mol) Benzotrichlorid zugetropft. Dann wird 1 Stunde bei gleicher Temperatur nachgerührt. Das Produktgemisch wird bei 10 mbar über eine verspiegelte Kolonne (3 cm × 30 cm) fraktioniert destilliert.

Die Ausbeuten sind in Tabelle 2 angegeben.

Tabelle 2

| Beispiel | 3 | 4 |
|---|---|---|
| Reaktionstemp. | 60° | 110° |
| $Ph-SO_2Cl$ (%) | 21 | 72 |
| Ph-COCl (%) | 67 | 86 |

*Beispiele 5 bis 8*

Die Beispiele 5 bis 8 wurden in gleicher Ansatzgrösse analog Beispiel 3 durchgeführt, jedoch wird der Benzolsulfonsäure jeweils eine definierte Menge Katalysator zugesetzt. Die Daten sind der nachstehenden Tabelle 3 zu entnehmen.

Tabelle 3

| Beispiel | Katalysator | Menge | Temp. | $PhSO_2Cl$ | PhCOCl |
|---|---|---|---|---|---|
| 5 | $FeCl_3$ | 1,0 g | 60° | 93% | 98% |
| 6 | $H_2SO_4$ | 7,9 g | 60° | 84% | 96% |
| 7 | $H_2SO_4$ | 7,9 g | 110° | 87% | 96% |
| 8 | $H_2SO_4$ | 4,0 g | 60° | 85% | 96% |

*Beispiel 9*

2500 g (14,8 Mol) einer 93,8%igen Benzolsulfonsäure, die durch Sulfonierung von Benzol mit $SO_3$ hergestellt wurde und 1,8 Gew.-% $H_2SO_4$ enthält, werden bei 110°C mit 3093 g (15,6 Mol) Benzotrichlorid (98,5%ig) analog Beispiel 4 umgesetzt.

Die Destillation erfolgt bei 10 mbar über eine 1-m-Spiegelkolonne (Durchmesser 5 cm, Füllung: Raschigringe 5 × 5 mm).

| Fraktion | Menge (g) | Gehalt (%) Benzoylchlorid | Benzotrichlorid | Benzolsulfochlorid |
|---|---|---|---|---|
| 1 | 1954,3 | 99,5 | — | — |
| 2 | 275,9 | 38,6 | 5,7 | 52,3 |
| 3 | 2114,5 | — | — | 99,5 |

Ausbeute: Benzoylchlorid 93,6%
Benzolsulfochlorid 85,9%

*Beispiel 10*

158 g (1 Mol) Benzolsulfonsäure (98,5%ig) und 1,0 g $FeCl_3$ werden bei 95°C analog Beispiel 3 mit 97,8 g (0,5 Mol) Benzotrichlorid (98,5%ig) umgesetzt.

Ausbeute: Benzolsulfochlorid 80%
Benzoesäure 76%

*Beispiel 11*

172 g (1 Mol) p-Toluolsulfonsäure (90%ig) und 4 g Schwefelsäure werden bei 80°C analog Beispiel 3 mit 207 g (1,06 Mol) Benzotrichlorid umgesetzt.

Ausbeute: p-Toluolsulfochlorid 93,6%
Benzoylchlorid 97,1%
Benzotrichlorid 2,2%

*Beispiel 12*

158 g (1 Mol) Benzolsulfonsäure (98,5%ig) und 4 g Schwefelsäure werden analog Beispiel 4 mit 166 g (0,53 Mol) 1,3-Bis-(trichlormethyl)-benzol umgesetzt.

Ausbeute: Benzolsulfochlorid 94,3%
Isophthaloylchlorid 92,1%
1,3-Bis-(trichlormethyl)-benzol 2,8%

*Beispiel 13*

841,5 g (5 Mol) einer 94%igen Benzolsulfonsäure, die durch Sulfonierung von Benzol mit $SO_3$ hergestellt wurde und 1,8 Gew.-% $H_2SO_4$ enthält, werden bei 95°C mit 977,5 g (5 Mol) Benzotrichlorid (98,5%ig) innerhalb 1 Stunde versetzt. Dann wird 1 Stunde bei 95°C nachgerührt. Die Destillation erfolgt bei 10 mbar über eine verspiegelte Kolonne (3 cm × 30 cm).

Ausbeute: Benzoylchlorid 96,3%
Benzolsulfochlorid 87,8%

**Patentansprüche**

1. Verfahren zur gleichzeitigen Herstellung aromatischer Sulfonsäurehalogenide und aromatischer Carbonsäurehalogenide oder der zugehörigen Carbonsäuren durch Umsetzung aromatischer Sulfonsäuren oder Sulfonsäureanhydriden mit aromatischen Trihalogenmethyl-Verbindungen, dadurch gekennzeichnet, dass man mindestens ein halbes Äquivalent einer aromatischen Trihalogenmethyl-Verbindung pro Äquivalent Sulfonsäure in Gegenwart einer Brönsted- oder Lewis-Säure bei einer Temperatur von 30 bis 130°C umsetzt.

2. Verfahren nach Anspruch 1, dadurch gekennzeichnet, dass man zur Herstellung aromatischer Sulfonsäurechloride als aromatische Trihalogenmethyl-Verbindung die Trichlormethyl-Verbindung einsetzt.

3. Verfahren nach Anspruch 1, dadurch gekennzeichnet, dass man als Brönsted- oder Lewis-Säure Schwefelsäure, Chlorsulfonsäure, Eisen-(III)-chlorid oder Zinkchlorid einsetzt.

4. Verfahren nach Anspruch 1, dadurch gekennzeichnet, dass man in Gegenwart von 0,01 bis 20 Mol-%, bezogen auf die aromatische Sulfonsäure, einer Brönsted- oder Lewis-Säure arbeitet.

5. Verfahren nach Anspruch 1 bis 4, dadurch gekennzeichnet, dass man zur Herstellung des aromatischen Sulfonsäurehalogenids und der simultanen Herstellung des der aromatischen Trihalogenmethyl-Verbindung entsprechenden aromatischen Carbonsäurehalogenids eine mindestens äquivalente Menge der aromatischen Trihalogenmethyl-Verbindung, bezogen auf die aromatische Sulfonsäure, einsetzt.

6. Verfahren nach Anspruch 1 bis 4, dadurch gekennzeichnet, dass man zur Herstellung des aromatischen Sulfonsäurehalogenids und der simultanen Herstellung der zur eingesetzten aromatischen Trihalogenmethyl-Verbindung korrespondierenden aromatischen Carbonsäure eine halbäquivalente Menge der aromatischen Trihalogenmethyl-Verbindung, bezogen auf die aromatische Sulfonsäure, einsetzt.

7. Verfahren nach Anspruch 1 bis 6, dadurch gekennzeichnet, dass man als aromatische Sulfonsäuren solche der allgemeinen Formel

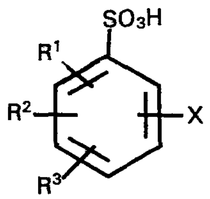

eingesetzt werden, in der

X für Wasserstoff, 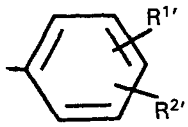

oder -Y- 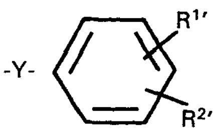

steht, wobei Y für -O-, $-SO_2-$, -N=N- oder $-CH_2-$ steht, und

$R^1$, $R^2$, $R^3$, $R^{1'}$ und $R^{2'}$ unabhängig voneinander Wasserstoff, Alkyl, Cycloalkyl, Aryl, Aralkyl, Hydroxy, Alkoxy, Alcyloxy, Halogen, Isocyanato, Acylamino, N-Carbalkoxyamino, N-Carbaryloxyamino, Nitro, die Sulfonsäuregruppe, die Carbonsäure, die Carbonsäureestergruppe oder die Harnstoffgruppe bedeuten, und weiterhin zwei der Reste $R^1$, $R^2$ oder $R^3$, wenn sie benachbart sind, Teil eines kondensierten, gegebenenfalls durch eine Sulfonsäuregruppe substituierten cycloaliphatischen oder aromatischen Ringes sein können.

8. Verfahren nach Anspruch 1 bis 7, dadurch gekennzeichnet, dass anstelle der aromatischen Sulfonsäure nach Anspruch 7 das entsprechende Sulfonsäureanhydrid eingesetzt wird.

9. Verfahren nach Anspruch 1 bis 8, dadurch gekennzeichnet, dass als aromatische Trihalogenmethyl-Verbindungen solche der allgemeinen Formel

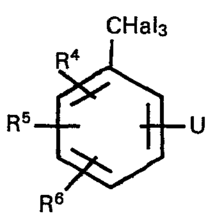

eingesetzt werden, in der

U für Wasserstoff, 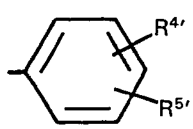

oder -V- 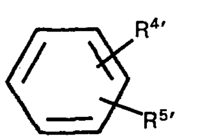

steht, wobei V für -O-, $-SO_2-$, -N=N- oder $-CH_2-$ steht, und

$R^4$, $R^5$, $R^6$, $R^{4'}$ und $R^{5'}$ unabhängig voneinander Wasserstoff, Alkyl, Cycloalkyl, Aryl, Aralkyl, Alkoxy, Aryloxy, $CHal_3$, Halogen, Isocyanato, N-Carbalkoxyamino oder N-Carbaryloxyamino bedeuten und weiterhin zwei der Reste $R^4$, $R^5$ oder $R^6$, wenn sie benachbart sind, Teil eines kondensierten, gegebenenfalls durch $CHal_3$ substituierten cycloaliphatischen oder aromatischen Ringes sein können und Hal für Chlor oder Brom steht.

**Claims**

1. Process for the simultaneous preparation of aromatic sulphonic acid halides and aromatic carboxylic acid halides or of the corresponding carboxylic acids by reaction of aromatic sulphonic acids or sulphonic acid anhydrides with aromatic trihalogenomethyl compounds, characterised in that at least half an equivalent of an aromatic trihalogenomethyl compound is reacted per equivalent of sulphonic acid, in the presence of a Brönsted acid or Lewis acid at a temperature of 30 to 130°C.

2. Process according to Claim 1, characterised in that the trichloromethyl compound is employed as the aromatic trihalogenomethyl compound for the preparation of aromatic sulphonic acid chlorides.

3. Process according to Claim 1, characterised in that sulphuric acid, chlorosulphonic acid, iron-III chloride or zinc chloride is employed as the Brönsted acid or Lewis acid.

4. Process according to Claim 1, characterised in that it is carried out in the presence of 0.01 to 20 mol %, relative to the aromatic sulphonic acid, of a Brönsted acid or Lewis acid.

5. Process according to Claim 1 to 4, characterised in that at least an equivalent amount of the aromatic trihalogenomethyl compound, relative to the aromatic sulphonic acid, is employed for the preparation of the aromatic sulphonic acid halide and the simultaneous preparation of the aromatic carboxylic acid halide corresponding to the aromatic trihalogenomethyl compound.

6. Process according to Claim 1 to 4, characterised in that half the equivalent amount of the aromatic trihalogenomethyl compound, relative to the aromatic sulphonic acid, is employed for the preparation of the aromatic sulphonic acid halide and for the simultaneous preparation of the aromatic carboxylic acid corresponding to the aromatic trihalogenomethyl compound employed.

7. Process according to Claim 1 to 6, characterised in that the aromatic sulphonic acids used are those of the general formula

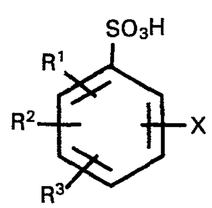 (I)

in which

X represents hydrogen, 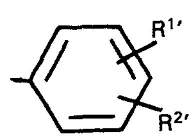

or -Y- 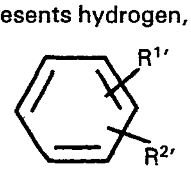

wherein Y represents -O-, $-SO_2-$, -N=N- or $-CH_2-$, and

$R^1$, $R^2$, $R^3$, $R^{1'}$ and $R^{2'}$ independently of one another denote hydrogen, alkyl, cycloalkyl, aryl, aralkyl, hydroxyl, alkoxy, acyloxy, halogen, isocyanato, acylamino, N-carbalkoxyamino, N-carbaryloxyamino, nitro, the sulphonic acid group, the carboxylic acid, the carboxylic acid ester group or the urea group, and, furthermore,

two of the radicals $R^1$, $R^2$ or $R^3$, if they are adjacent, can be a part of a fused-on cycloaliphatic or aromatic ring which is optionally substituted by a sulphonic acid group.

8. Process according to Claim 1 to 7, characterised in that instead of the aromatic sulphonic acid according to Claim 7 the corresponding sulphonic acid anhydride is employed.

9. Process according to Claim 1 to 8, characterised in that the aromatic trihalogenomethyl compounds used are those of the general formula

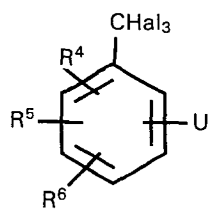

in which

U represents hydrogen, 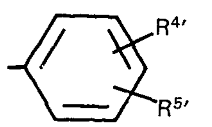

or -V- 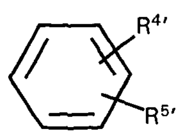

wherein V represents -O-, $-SO_2-$, -N=N- or $-CH_2-$, and

$R^4$, $R^6$, $R^{4'}$ and $R^{5'}$ independently of one another denote hydrogen, alkyl, cycloalkyl, aryl, aralkyl, alkoxy, aryloxy, $CHal_5$, halogen, isocyanato, N-carbalkoxyamino or N-carbaryloxyamino and, furthermore,

two of the radicals $R^4$, $R^5$ or $R^6$, if they are adjacent, can be a part of a fused-on cycloaliphatic or aromatic ring which is optionally substituted by $CHal_3$, and

Hal represents chlorine or bromine.

**Revendications**

1. Procédé pour la préparation simultanée d'halogénures d'acides sulfoniques aromatiques et d'halogénures d'acides carboxyliques aromatiques ou des acides carboxyliques correspondants par réaction d'acides sulfoniques aromatiques ou d'anhydrides d'acides sulfoniques avec des composés trihalogénométhyle aromatiques, caractérisé en ce qu'on fait réagir au moins un demi-équivalent d'un composé trihalogénométhyle aromatique par équivalent d'acide sulfonique en présence d'un acide de Brönsted ou d'un acide de Lewis à une température de 30 à 130°C.

2. Procédé suivant la revendication 1, caractérisé en ce que, pour la préparation de chlorures d'acides sulfoniques aromatiques, comme composé trihalogénométhyle aromatique, on utilise le composé trichlorométhyle.

3. Procédé suivant la revendication 1, caractérisé en ce que, comme acide de Brönsted ou acide de Lewis, on utilise l'acide sulfurique, l'acide chlorosulfonique, le chlorure de fer(III) ou le chlorure de zinc.

4. Procédé suivant la revendication 1, caractérisé en ce qu'on travaille en présence de 0,01 à 20% molaires (calculé sur l'acide sulfonique aromatique) d'un acide de Brönsted ou d'un acide de Lewis.

5. Procédé suivant les revendications 1 à 4, caractérisé en ce que, pour la préparation de l'halogénure d'acide sulfonique aromatique et pour la préparation simultanée de l'halogénure d'acide carboxylique aromatique correspondant au composé trihalogénométhyle aromatique, on utilise une quantité au moins équivalente du composé trihalogénométhyle aromatique, calculé sur l'acide sulfonique aromatique.

6. Procédé suivant les revendications 1 à 4, caractérisé en ce que, pour la préparation de l'halogénure d'acide sulfonique aromatique et pour la préparation simultanée de l'acide carboxylique aromatique correspondant au composé trihalogénométhyle aromatique employé, on utilise une quantité semi-équivalente du composé trihalogénométhyle aromatique, calculé sur l'acide sulfonique aromatique.

7. Procédé suivant les revendications 1 à 6, caractérisé en ce que, comme acides sulfoniques aromatiques, on utilise ceux répondant à la formule générale:

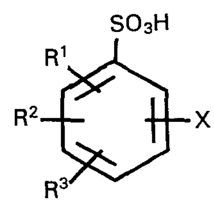

dans laquelle

X représente un atome d'hydrogène, un groupe

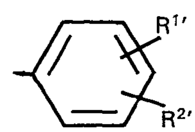 ou un groupe -Y- 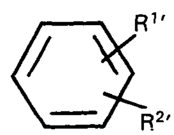

groupe alcoxy, un groupe acyloxy, un atome d'halogène, un groupe isocyanato, un groupe acylamino, un groupe N-carbalcoxyamino, un groupe N-carbaryloxyamino, un groupe nitro, le groupe d'acide sulfonique, l'acide carboxylique, le groupe d'ester d'acide carboxylique ou le groupe urée et, en outre, lorsqu'ils sont voisins, deux des radicaux $R^1$, $R^2$ et $R^3$ peuvent faire partie d'un noyau aromatique ou cycloaliphatique condensé et éventuellement substitué par un groupe d'acide sulfonique.

8. Procédé suivant les revendications 1 à 7, caractérisé en ce que, au lieu de l'acide sulfonique aromatique, on utilisé l'anhydride d'acide sulfonique correspondant.

9. Procédé suivant les revendications 1 à 8, caractérisé en ce que, comme composés trihalogénométhyle aromatiques, on utilise ceux répondant à la formule générale:

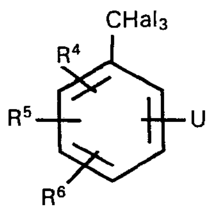

dans laquelle

U représente un atome d'hydrogène, un groupe

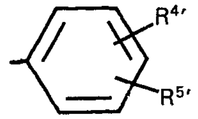

ou un groupe -V-

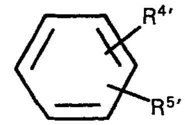

V représentant -O-, $-SO_2-$, -N = N- ou $-CH_2-$, et

$R^4$, $R^6$, $R^{4'}$ et $R^{5'}$ représentent chacun indépendamment lun de l'autre un atome d'hydrogène, un groupe alkyle, un groupe cycloalkyle, un groupe aryle, un groupe aralkyle, un groupe alcoxy, un groupe aryloxy, $CHal_3$, un atome d'halogène, un groupe isocyanato, un groupe N-carbalcoxyamino ou un groupe N-carbaryloxyamino et, en outre, lorsqu'ils sont voisins, deux des radicaux $R^4$, $R^5$ et $R^6$ peuvent faire partie d'un noyau aromatique ou cycloaliphatique condensé et éventuellement substitué par $CHal_3$, et

Hal représente un atome de chlore ou un atome de brome.